# EUROPÄISCHE PATENTSCHRIFT

(11) **EP 2 388 588 B1**
(45) Veröffentlichungstag und Bekanntmachung des Hinweises auf die Patenterteilung: **30.10.2013**
(21) Anmeldenummer: 10163147.1
(22) Anmeldetag: 18.05.2010
(51) Int. Cl.: G01N 33/50, G01N 33/558, G01N 33/68

(54) **Verfahren und Schnelltest zur Bestimmung der Fertilität von Spermien**
Method and quick test for determining the fertility of sperms
Procédé et test rapide destinés à la détermination de la fertilité de spermatozoïdes

(43) Veröffentlichungstag der Anmeldung: 23.11.2011
(73) Patentinhaber: Justus-Liebig-Universität Gießen, 35390 Giessen (DE)
(72) Erfinder: Steger, Klaus, 35392 Gießen (DE); Paradowska, Agnieszka, 35039 Marburg (DE); Bärmann, Birte, 35392 Gießen (DE)
(74) Vertreter: Patentanwälte Olbricht Buchhold Keulertz

(56) Entgegenhaltungen:
- WO-A1-98/36278
- WO-A2-96/13225
- US-A1- 2006 008 923
- STEGER KLAUS ET AL: "Round spermatids from infertile men exhibit decreased protamine-1 and 2 mRNA" HUMAN REPRODUCTION (OXFORD), Bd. 16, Nr. 4, April 2001 (2001-04), Seiten 709-716, XP002593072 ISSN: 0268-1161
- NASR-ESFAHANI M H ET AL: "Effect of protamine-2 deficiency on ICSI outcome." REPRODUCTIVE BIOMEDICINE ONLINE DEC 2004 LNKD- PUBMED:15670415, Bd. 9, Nr. 6, Dezember 2004 (2004-12), Seiten 652-658, XP027052356 ISSN: 1472-6483
- AOKI V W ET AL: "Sperm protamine 1/protamine 2 ratios are related to in vitro fertilization pregnancy rates and predictive of fertilization ability" FERTILITY AND STERILITY, ELSEVIER SCIENCE INC, NEW YORK, NY, USA LNKD- DOI:10.1016/J.FERTNSTERT.2006.04.024, Bd. 86, Nr. 5, 1. November 2006 (2006-11-01), Seiten 1408-1415, XP025233244 ISSN: 0015-0282 [gefunden am 2006-11-01]
- AOKI V W ET AL: "A novel mechanism of protamine expression deregulation highlighted by abnormal protamine transcript retention in infertile human males with sperm protamine deficiency" MOLECULAR HUMAN REPRODUCTION, Bd. 12, Nr. 1, Januar 2006 (2006-01), Seiten 41-50, XP002593073 ISSN: 1360-9947
- DATABASE EMBASE [Online] ELSEVIER SCIENCE PUBLISHERS, AMSTERDAM, NL; Dezember 2005 (2005-12), SALEHI M ET AL: "The relationship between sperm protamine aberrations with protamineP1/protamineP2 ratio" XP002593103 Database accession no. EMB-2005189853
- STANKER LARRY H ET AL: "Immunological evidence for a P2 protamine precursor in mature rat sperm" MOLECULAR REPRODUCTION AND DEVELOPMENT, Bd. 33, Nr. 4, 1992, Seiten 481-488, XP002593075 ISSN: 1040-452X

## Beschreibung

Die vorliegende Erfindung betrifft ein Verfahren und einen Schnelltest zur Bestimmung der Fertilität von Spermien von Säugetieren, vorzugsweise von menschlichen Spermien. Die Bestimmung der Fertilität von Spermien erfolgt durch Nachweis der Proteine Protamin-1 und Protamin-2 und die Darstellung des Verhältnisses zwischen Protamin-1 und Protamin-2 (Protamin-Protein-Ratio). Die Protamin-Protein-Ratio korreliert mit der Fertilität von Spermien.

Das Verfahren und der Schnelltest werden verwendet, um eine Prognose für den Erfolg einer künstlichen Befruchtung einer Eizelle zu erhalten.

### Abkürzungen und Definitionen

DNA: Deoxyribonucleic Acid, Desoxyribonukleinsäure
ELISA: Enzyme-linked Immunosorbent Assay, Enzymgekoppelter Immunadsorptionstest
Fragliche Probe: Die fragliche Probe ist biologisches Material eines männlichen Probanden, das zur Bestimmung der Fertilität von Spermien geeignet ist. Die fragliche Probe ist insbesondere Ejakulat, Hodengewebe oder Nebenhodengewebe, die Spermien oder Spermatiden enthält. Zur Bestimmung der Fertilität erfolgt ein Nachweis der Proteine Protamin-1 und Protamin-2 aus der fraglichen Probe und die Darstellung des Verhältnisses zwischen Protamin-1 und Protamin-2 (Protamin-Protein-Ratio), welches mit der Fertilität von Spermien korreliert.
Histone: Proteine im Zellkern von Eukaryoten, die als Bestandteil des Chromatins für die Verpackung der DNA eine wesentliche Rolle spielen.
ICSI: Intracytoplasmatic Sperm Injection, Intrazytoplasmatische Spermieninjektion; Verfahren zur künstlichen Befruchtung einer Eizelle.
IU: International Unit, Internationale Einheit
IVF: In-vitro-Fertilisation; Verfahren zur künstlichen Befruchtung einer Eizelle.
mL: Milliliter
mM: Millimol
mRNA: Messenger RNA, Boten-RNA; RNA-Transkript eines zu einem Gen gehörigen Teilabschnittes der DNA.
Nukleinsäuresequenz: Abfolge der Nukleotide einer beliebigen Nukleinsäure, beispielsweise DNA oder RNA.
P-1: Protein Protamin-1
P-2: Protein Protamin-2
PCR: Polymerase Chain Reaction, Polymerase-Kettenreaktion; Verfahren zur Vervielfältigung von Nukleinsäuresequenzen in vitro.
Protamine: Argininreiche Proteine im Zellkern von Spermien von Säugetieren (inklusive Mensch), welche die Histone in der späten haploiden Phase der Spermatogenese ersetzen.
Protamin-mRNA-Ratio: Verhältnis der Menge der mRNA von Protamin-1 zur Menge der mRNA von Protamin-2 in einer fraglichen Probe.
Protamin-Protein-Ratio: Verhältnis der Menge von Protamin-1 zur Menge von Protamin-2 in einer fraglichen Probe. Eine Protamin-Protein-Ratio von 1 zu 1 gilt erfindungsgemäß als Hinweis auf positive Fertilität der Spermien oder Spermatiden in der fraglichen Probe. Erfindungsgemäß gilt auch der Grenzbereich 0,8 bis 1,2 in der Protamin-Protein-Ratio als Hinweis auf positive Fertilität der Spermien oder Spermatiden in der fraglichen Probe.
RNA: Ribonucleic Acid, Ribonukleinsäure
rpm: rounds per minute, Umdrehungen pro Minute
Spermatoaenese: Vermehrung und Differenzierung der männlichen Keimzellen in den Hoden, wobei Spermien entstehen.
Spermatiden: Vorgängerzellen von Spermien
TESE: Testikuläre Spermienextraktion; Entnahme von Gewebe aus dem Hoden um Spermatiden oder Spermien zu gewinnen.

### Beschreibung des allgemeinen Gebietes der Erfindung

Für die Befruchtung einer Eizelle sind fertile Spermien erforderlich. Da bei unerfülltem Kinderwunsch eine mangelhafte Fertilität der Spermien eine wesentliche Rolle spielen kann, ist die Diagnostik der Fertilität von Spermien in der Human- und Veterinärmedizin von großer Bedeutung. Vor allem beim Einsatz von Verfahren zur künstlichen Befruchtung einer Eizelle ist die vorhergehende Bestimmung der Fertilität der eingesetzten Spermien essentiell.

Während der Spermatogenese werden DNA-bindende Histone gegen Protamine ausgetauscht. Beim Menschen kommen zwei verschiedene Protamine vor, die als Protamin-1 (P-1) und Protamin-2 (P-2) bezeichnet werden. Der Nachweis von Protaminen kann an Hodengewebsproben oder an Ejakulat durchgeführt werden. Der qualitative Nachweis von Protaminen in Hodengewebsproben zeigt die Anwesenheit von Spermien an und dient als Prognosefaktor für eine testikuläre Spermienextraktion (TESE).

Der quantitative Nachweis von Protaminen in Hodengewebsproben oder Ejakulaten gibt Auskunft über die Fertilisierungswahrscheinlichkeit der vorhandenen Spermien. Überraschenderweise ist vor allem das Verhältnis von Protamin-1 zu Protamin-2 (beispielsweise indirekt bestimmt als Protamin-mRNA-Ratio) ein valider Prognosemarker für den Erfolg einer nachfolgenden In-vitro-Fertilisation oder intrazytoplasmatischen Spermieninjektion. Der Nachweis von Protaminen zur Bestimmung der Fertilität von Spermien ist spezifisch, da Protamine nur in männlichen haploiden Keimzellen (und damit auch in Spermien) vorkommen. Zur Bestimmung der Fertilität von Spermien, vor allem von humanen Spermien, sind verschiedene Verfahren im Stand der Technik bekannt.

### Stand der Technik

Der Stand der Technik kennt zur Bestimmung der Fertilität von Spermien vor allem die Ejakulatanalyse, z.B. nach den Kriterien der WHO. Dabei wird unter anderem die absolute Spermienzahl pro Volumeneinheit bestimmt, sowie die Morphologie und Beweglichkeit der Spermien. Nachteilig ist, dass aus dieser Ejakulatanalyse keine Prognosefaktoren für den Erfolg einer künstlichen Befruchtung einer Eizelle ableitbar sind. Die Durchführung des Verfahrens erfordert spezielle Laborausstattung und kann nur von ausgebildetem Fachpersonal durchgeführt werden.
Ein weiteres Verfahren ist die histologische Untersuchung einer Hodengewebsprobe, wobei Vorhandensein und Morphologie von Spermien im Hodengewebe beurteilt werden. Nachteilig ist, dass mit dieser histologischen Untersuchung keine Aussage möglich ist, ob die vorhandenen Spermien funktionell intakt sind, was oft nicht der Fall ist, auch wenn die Morphologie der Spermien dies nahelegen würde. Eine verlässliche Beurteilung der Fertilitätswahrscheinlichkeit der vorhandenen Spermien oder eine Prognose zum Erfolg einer künstlichen Befruchtung einer Eizelle ist mit diesem Verfahren nicht möglich. Zudem hängt die Beurteilung der Morphologie der Spermien sehr stark von der Erfahrung des Untersuchers ab und ist deshalb nicht standardisierbar. Die Durchführung des Verfahrens erfordert spezielle Laborausstattung und geschultes Fachpersonal und ist nicht von jedermann an einem beliebigen Ort durchführbar.

Die WO 96/13225 A2 befasst sich mit Analysen, Vorrichtungen und Kitsystemen für die Bestimmung der männlichen Fertilität, wobei - je nach Anwendungszweck - die Identifizierung von Spermaproben mit hohem oder geringem Befruchtungspotential vorgesehen ist. Dazu erfolgt beispielsweise die Quantifizierung von Spermien in einer Spermaprobe mit Hilfe eines Teststreifens, welcher einen Antikörper, beispielsweise für Protamin, enthalten kann.

Die WO 98/36278 A1 beschäftigt sich mit Vorrichtungen, Analysen und Kitsystemen für die Quantifizierung eines Analyten in einer Probe, wobei die Vorrichtungen eine Vielzahl von Detektionszonen für das Detektieren und Quantifizieren des Analyten beinhalten. Für die Analyse von Spermaproben wird beispielsweise Protamin als Targetmolekül verwendet.

Stanker et al. (Molecular Reproduction and Development 1992, 33, 481 - 488) befassten sich mit dem immunologischen Nachweis eines Protamin-2-Päkursoren in Sperma der Wanderratte.

Nasr-Esfahani et al. (Reproductive Biomedicine 2004, 9, 652 - 658) beschrieben die Bestimmung der Protamin-Protein-Ratio mittels nuklearer Proteinextraktion, Gelelektrophorese und Software-basierter Analyse der Proteinbanden.

Salehi et al. (Cell Journal 2005, 6, 212 - 217) beschäftigten sich mit der Beziehung zwischen Sperma-Protamin-Anomalien und dem Protamin-1/Protamin-2-Verhältnis. Zur Bestimmung von Protamin-1 und Protamin-2 kam ein Western-Blot zum Einsatz.

Aoki et al. (Fertil. Steril. 2006, 86, 1408 - 1415; Molecular Human Reproduction 2006, 12, 41 - 50) befassten sich unter anderem mit der Bestimmung der Protamin-Protein-Ratio, wobei sie nukleare Proteinextraktion, Gelelektrophorese und Densitometrie einsetzten.

In der US 2006/008923 A1 sind "Point-of-care"-Diagnosesysteme beschrieben, d. h. Schnelltest-Diagnosesysteme, beispielsweise zur Bestimmung fötalen Fibronektins.

Ein anderer Ansatz zur Bestimmung der Fertilität von Spermien wurde durch die Erfinder selber offenbart (Steger et al., Human Reproduction 2001, 16, 709 - 716; Human Reproduction 2007, 23, 11 - 16). Dieses Verfahren basiert auf einem mRNA-Nachweis, insbesondere dem Nachweis von mRNA von Protamin-1 und dem Nachweis von mRNA von Protamin-2, die in ein Verhältnis, die sogenannte Protamin-mRNA-Ratio, gebracht werden. Die Protamin-mRNA-Ratio gibt Hinweise auf die Fertilität von Spermien. Auch dieses Verfahren hat große Nachteile. Es ist ein sehr spezielles Laborverfahren, das aufgrund der Empfindlichkeit der RNA und deren schnellen Degradierung besondere Reinheit bei der Isolierung und dem Umgang erfordert und nur von sehr gut ausgebildetem Fachpersonal mit speziellen PCR-Geräten in reproduzierbarer Qualität durchgeführt werden kann. Das Verfahren ist sehr fehleranfällig, dabei vergleichsweise zeitaufwendig und teuer, insgesamt nicht für eine Routinemethode geeignet, die von jedermann an einem beliebigen Ort durchgeführt werden kann.

### Aufgabe

Aufgabe der Erfindung ist daher die Bereitstellung eines schnell durchführbaren, standardisierbaren und kostengünstigen Verfahrens zur zuverlässigen Bestimmung der Fertilität von Spermien, das von jedermann ohne spezielle Kenntnisse und ohne eine aufwendige Laborausstattung an einem beliebigen Ort durchgeführt werden kann sowie eines Schnelltestes, mit dem das Verfahren durchgeführt wird.

### Lösung

Die Aufgabe wird erfindungsgemäß durch ein immunologisches Verfahren zur Bestimmung der Fertilität männlicher Säugetiere und Menschen gelöst, wobei die Konzentration an Protamin-1 und Protamin-2 und das Verhältnis zwischen Protamin-1 und Protamin-2, die Protamin-Protein-Ratio, in einer fraglichen Probe eines zu untersuchenden Individuums *in vitro* ermittelt wird, wobei die Konzentration an Protamin-1 und Protamin-2 und die Protamin-Protein-Ratio mittels Schnelltest auf einem Teststreifen ermittelt wird, wobei der Schnelltest auf dem Teststreifen einen Spermienvortest und einen Protamin-Schnelltest umfasst.

Ferner wird die Aufgabe durch einen Teststreifen zur Durchführung des erfindungsgemäßen Verfahrens gelöst, wobei der Teststreifen eine Grundfläche und eine Auftragschicht umfasst, wobei auf der Auftragschicht eine Zone V zur Durchführung des Spermienvortestes zur Ermittlung der Konzentration der Spermien vorhanden ist, ferner eine Zone P1 die einen für Protamin-1 spezifischen Antikörper enthält zur Ermittlung der Konzentration von Protamin-1 und eine Zone P2 die einen für Protamin-2 spezifischen Antikörper enthält zur Ermittlung der Konzentration von Protamin-2.

Überraschenderweise wurde diagnostisch gefunden und medizinisch nachgewiesen, dass in Proben mit Spermien oder Spermatiden gesunder Männer eine Protamin-Protein-Ratio, also ein quantitatives Verhältnis von Protamin-1 zu Protamin-2 von 1 zu 1 eine normale Fertilität der Spermien anzeigt.
Dies gilt sogar auch in einem Toleranzbereich, wenn in den Proben mit Spermien oder Spermatiden die Protamin-Protein-Ratio statt 1 zu 1 dann 0,8 bis 1,2 Protamin-1 zu 0,8 bis 1,2 Protamin-2 ist. Liegt dagegen in Proben mit Spermien oder Spermatiden eine davon abweichende Protamin-Protein-Ratio vor, so weist dies auf eine verminderte Fertilität bis hin zu Infertilität der Spermien oder Spermatiden in der fraglichen Probe hin. Diese Spermien werden dann nicht für eine nachfolgende in-vitro-Fertilisation oder intrazytoplasmatische Spermieninjektion verwendet. Zahlreiche experimentelle Analysen der Erfinder mit verschiedenen fraglichen Proben von humanen Säugern dokumentieren, dass die Protamin-Protein-Ratio, also das quantitative Verhältnis von Protamin-1 zu Protamin-2 ein valider Prognosemarker für den Erfolg einer nachfolgenden in-vitro-Fertilisation oder intrazytoplasmatischen Spermieninjektion ist.
Die Ermittlung der Protamin-Protein-Ratio in Spermien oder Spermatiden erfolgt erfindungsgemäß in einem speziellen Verfahren und mittels Schnelltest.

Dazu wird eine fragliche Probe in einem geeigneten Verfahren so aufbereitet, dass die Proteine und insbesondere Protamin-1 und Protamin-2 aus den Spermien oder Spermatiden isoliert werden, damit sie frei zugänglich sind und separat immunologisch nachgewiesen werden können. Dann erfolgt der qualitative und quantitative Nachweis von Protamin-1 und von Protamin-2 und die Ermittlung der Protamin-Protein-Ratiomittelseines Schnelltests.

Der untere Grenzwert der Protaminkonzentrationen beim Schnelltest wurde mittels des Protamin-ELISAs auf 0,2 Mio. Spermien/mL festgelegt. Für die Durchführung des ELISA-Verfahrens kommt die aufbereitete Probe in einem Ansatz in Kontakt mit einem Antikörper (anti-Protamin-1-Antikörper), dessen Epitop spezifisch gegen Protamin-1 gerichtet ist, in einem anderen Ansatz in Kontakt mit einem Antikörper (anti-Protamin-2-Antikörper), dessen Epitop spezifisch gegen Protamin-2 gerichtet ist. Ist Protamin-1 und/oder Protamin-2 in der fraglichen Probe vorhanden, kommt es zu einer Bindung zwischen Protamin-1 und dem korrespondierendem anti-Protamin-1-Antikörper und/oder zwischen Protamin-2 und dem korrespondierendem anti-Protamin-2-Antikörper. Diese Bindungen werden durch Kontakt mit einem Sekundärantikörper, dessen Epitop an anti-Protamin-1-Antikörper und anti-Protamin-2-Antikörper spezifisch bindet, durch eine Farbreaktion sichtbar gemacht und mit geeigneten Geräten nachgewiesen und quantifiziert.
Die Sichtbarmachung der Bindung durch den Sekundärantikörper erfolgt durch eine enzymatische oder physikalische Farbreaktion.

Die Intensität der Farbreaktion in den Ansätzen von Protamin-1 und Protamin-2 wird ermittelt und zueinander ins Verhältnis gesetzt, so dass sich die Protamin-Protein-Ratio (Verhältnis der Menge von Protamin-1 zu der Menge von Protamin-2) ergibt. Diese Protamin-Protein-Ratio ist ein valider Prognosemarker für den Erfolg einer nachfolgenden in-vitro-Fertilisation oder intrazytoplasmatischen Spermieninjektion. Liegt die Protamin-Protein-Ratio in Spermien oder Spermatiden einer fraglichen Probe bei 0,8 bis 1,2 Protamin-1 zu 0,8 bis 1,2 Protamin-2, vorzugsweise bei 1 zu 1, dann liegt eine normale Fertilität der Spermien vor und sie können für eine nachfolgende in-vitro-Fertilisation oder intrazytoplasmatische Spermieninjektion verwendet werden. Liegt dagegen ein davon abweichendes Verhältnis vor, so weist dies auf eine verminderte Fertilität bis hin zu Infertilität der Spermien oder Spermatiden in der fraglichen Probe hin. Diese Spermien werden nicht für eine nachfolgende in-vitro-Fertilisation oder intrazytoplasmatische Spermieninjektion verwendet.

Dieses Verfahren ermöglicht erstmals die schnelle, zuverlässige, standardisierbare und kostengünstige Bestimmung der Fertilität von Spermien und stellt eine wesentliche Verbesserung der Bestimmung der Fertilität von Spermien und Spermatiden in einer fraglichen Probe dar.

Die Aufbereitung einer fraglichen Probe umfasst folgende Schritte:
- Gewinnung und Aufkonzentrierung der Spermien aus einer fraglichen Probe
- Dekondensation der dichten Verbindungen von DNA, Histonen und Protaminen in den Spermien, so dass die Proteine insbesondere Protamin-1 und/oder Protamin-2 freigesetzt werden
- Zellaufschluss

Insbesondere erfolgt die Gewinnung und Aufkonzentrierung der Spermien aus einer fraglichen Probe wie z.B. aus Ejakulat, Nebenhodengewebe oder Hodengewebe z.B. durch Sedimentation oder Zentrifugation.

Die Dekondensation der dichten Verbindungen von DNA, Histonen und Protaminen in den Spermien einer fraglichen Probe erfolgt z.B. durch Inkubation mit einem geeigneten Puffer, der z.B. Detergenzien oder Enzyme enthält, so dass die Proteine insbesondere Protamin-1 und/oder Protamin-2 freigesetzt werden.

Alternativ erfolgt in der Probe eine Bestimmung des Gesamtproteingehaltes der dekondensierten Spermien, so dass eine bestimmte Proteinkonzentration eingestellt werden kann, beispielsweise nach einer dem Fachmann bekannten Methode z.B. mit dem Bradford-, Biuret- oder Lowry-Test.

Die angeführten Ausführungsbeispiele zeigen besonders vorteilhafte Ausführungsbeispiele, schränken den Inhalt der erfindungsgemäßen Lehre jedoch nicht ein.

### Ausführungsbeispiele

### 1. Aufbereitung einer fraglichen Probe

Die Gewinnung von Spermien aus einer fraglichen Probe z.B. einer Ejakulatprobe erfolgt z.B. durch Zentrifugation des gesamten Ejakulatvolumens (dieses variiert bei Menschen von 0,5 bis 6 mL) bei 2000 bis 5000 rpm in einem geeigneten Gefäß, so dass die Spermien konzentriert als Pellet vorliegen.
Die Dekondensation der dichten Verbindungen von DNA, Histonen und Protaminen in den Spermien erfolgt z.B. durch Resuspension des Spermien-Pellets mit Dekondensierungspuffer z.B. 25 mM DTT (1,4-Dithiothreitol; Fa. Roche), 0,2% TritonX-100 (Fa. Sigma), 200 IU Heparin/mL (Heparin-Natrium-5000-ratiopharm) in PBS (phosphate buffered saline) z.B. mit 2 mL Puffer, wobei die Inkubation so lange erfolgt, bis die Spermienköpfe zwar stark aufgequollen sind, der Spermienkern aber noch intakt ist. Dies wird z.B. durch mikroskopische Beobachtung ermittelt. Bevorzugt beträgt die Inkubationszeit 5 bis 30 min (unterscheidet sich je nach Ejakulatprobe). Danach wird die Suspension mit den dekondensierten Spermien gewaschen, z.B. indem sie mit Waschpuffer (z.B. PBS-Puffer mit einem Protease-Inhibitor) versetzt und vorzugsweise bis zu zweimal gewaschen wird. Bevorzugt werden dazu 100 bis 1000 µL Waschpuffer verwendet und jeweils zentrifugiert, bis die Spermien sedimentiert sind, z.B. 5 bis 15 min bei 500 bis 4000 rpm. Das Spermien-Pellet wird mit 100 bis 1000 µL Waschpuffer resuspendiert.
Anschließend erfolgt der Zellaufschluss, z.B. über Freeze-und-thaw-Zyklen. Anschließend wird die Suspension mit den dekondensierten Spermien z.B. 5 bis 20 min im Ultraschallbad und anschließend für 15 bis 60 sec auf Eis in einem Sonifikator bei 20 bis 60% Leistung behandelt.
Die Bestimmung des Gesamtproteingehaltes in der Suspension mit den dekondensierten Spermien erfolgt z.B. nach einer dem Fachmann bekannten Methode z.B. dem Bradford-, Biuret- oder Lowry-Test.
Die Suspension mit den dekondensierten Spermien wird anschließend mit einem Verdünnungspuffer (z.B. PBS-Puffer mit einem Protease-Inhibitor) auf eine geeignete Proteinkonzentration z.B. von 0,1 µg/mL, 0,25 µg/mL, 0,5 µg/mL, 1,0 µg/mL, 2,5 µg/mL, 5,0 µg/mL, 10,0 µg/mL und/oder 20 µg/mL eingestellt.

Für die Durchführung des Verfahrens im Schnelltest erfolgt die Aufbereitung einer fraglichen Probe, indem Ejakulat zur Verflüssigung 15 bis 60 min bei Raumtemperatur inkubiert wird und mit Dekondensationspuffer versetzt wird und 5 bis 30 min inkubiert wird, sodass eine Suspension mit dekondensierten Spermien entsteht. Der Zellaufschluss erfolgt durch Zugabe eines Lysepuffers z.B. 20 mM Tris-HCl (pH 7.5, 150 mM NaCl, 1 mM Na₂EDTA, 1 mM EGTA, 1 % Triton, 2,5 mM Sodium Pyrophosphat, 1 mM beta-Glycerophosphat, 1 mM Na₃VO₄, 1 µg/mL Leupeptin).
Alternativ wird eine unbehandelte fragliche Probe, z.B. Ejakulat verwendet.

Der obere Grenzwert der Protaminkonzentrationen beim Schnelltest ist darauf ausgelegt, dass selbst Ejakulatproben von z.B. 200 Mio. Spermien/mL gemessen werden können. Der untere Grenzwert der Protaminkonzentrationen wurde mittels des Protamin-ELISAs auf 0,2 Mio. Spermien/mL festgelegt.

### 2. Bestimmung der Konzentration von Protamin-1 und von Protamin-2 in einer fraglichen Probe

### 2.1. ELISA-Verfahren (indirektes Sandwich-Verfahren)

Ausgangsmaterial ist die aufbereitete fragliche Probe gemäß Ausführungsbeispiel 1, d.h. die Suspension mit dekondensierten Spermien mit einer eingestellten Proteinkonzentration von z.B. 0,1 µg/mL, 0,25 µg/mL, 0,5 µg/mL, 1,0 µg/mL, 2,5 µg/mL, 5,0 µg/mL, 10,0 µg/mL und/oder 20 µg/mL.

Das indirekte Sandwich-Verfahren ist ein Standard-Enzymimmunassay. Dazu werden die Proteine Protamin-1 und Protamin-2 aus der aufbereiteten fraglichen Probe gemäß Ausführungsbeispiel 1 auf einer festen Phase z.B. einer 96-well-Mikrotiterplatte so immobilisiert, dass sie möglichst homogen verteilt sind. Dazu werden protaminspezifische Antikörper (anti-Protamin-1-Antikörper oder anti-Protamin-2-Antikörper) hinzugegeben. Sie binden in der ersten Inkubationsphase an die Proteine Protamin-1 und Protamin-2. Dabei wird in zwei separaten Ansätzen gearbeitet, wobei im ersten Ansatz der fraglichen Probe anti-Protamin-1-Antikörper zugefügt wird, der Protamin-1 bindet und in einem zweiten Ansatz derselben fraglichen Probe anti-Protamin-2-Antikörper zugefügt wird, der Protamin-2 bindet.

Anschließend werden die Vertiefungen gewaschen und den gewaschenen leeren Vertiefungen wird enzymkonjugierter Sekundärantikörper, dessen Epitop an anti-Protamin-1-Antikörper und anti-Protamin-2-Antikörper spezifisch bindet, hinzugefügt, so dass sich während des zweiten Inkubationsschritts der Sekundärantikörper an die fixierten Protamin-Antikörper spezifisch anlagert. Ungebundene Moleküle, Proteine und Antikörper werden in einem weiteren Waschschritt aus den Vertiefungen entfernt. Den Vertiefungen wird Substrat hinzugefügt. Insbesondere Substrat, das vom Enzym des enzymkonjugierten Sekundärantikörpers in eine Farbreaktion umgesetzt werden kann, z.B. das chromogene Substrat TMB (Tetramethylbenzidin). Das an sich farblose Substrat geht durch das in den Vertiefungen gebundene Enzymkonjugat in eine Farbe, z.B. blaue Farbe über. Die Farbreaktion wird durch Zugabe einer Stopplösung so gestoppt, dass die blaue Farbe in z.B. eine gelbe Farbe übergeht. Die Intensität der gelben Farbreaktion wird spektrophotometrisch gemessen, z.B. bei 450 nm, wobei die Farbintensität proportional ist zur Konzentration der in den Vertiefungen gebundenen anti-Protamin-1-Antikörper und anti-Protamin-2-Antikörper.

Bevorzugt wird die aufbereitete fragliche Probe in einem Volumen von 100 µL/Vertiefung in eine Mikrotiterplatte pipettiert und so inkubiert, dass die Proteine Protamin-1 und Protamin-2 an die Oberfläche gebunden werden. Die Mikrotiterplatte wird ausgeklopft und mit Blockierungslösung (z.B. kommerziell erhältliche Blocking solution mit Casein) versetzt und ebenfalls inkubiert. Die Blockierungslösung dient zur Absättigung von freien Bindungsstellen auf der protamingebundenen Oberfläche der Mikrotiterplatte. So werden unspezifische Bindungen an der Oberfläche verhindert, der Hintergrund reduziert und die Sensitivität des Verfahrens verbessert. Die Mikrotiterplatte wird mit einer Waschlösung (z.B. kommerziell erhältliche Washing solution auf TRIS-Basis mit Tween und 350 mM NaCl, 10-fach Konzentrat) versetzt. Durch die Waschschritte werden ungebundene und überschüssige Komponenten entfernt, die sonst den ELISA stören würden. Monoklonale anti-Protamin-IgG-Antikörper (z.B. kommerziell erhältlich von SHAL Technologies) werden mit Puffer (z.B. kommerziell erhältlicher Sample Buffer, Candor) auf eine Konzentration von 1 µg/mL (1:2100) eingestellt. 100 µL/Vertiefung dieser Lösung werden in die Vertiefungen der Mikrotiterplatte pipettiert und inkubiert z.B. 90 min bei 37° C.
Danach wird die Mikrotiterplatte mit Waschpuffer (z.B. kommerziell erhältliche Washing solution) gewaschen, z.B. dreimal mit je 200 µL/Vertiefung Waschpuffer für je 10 sec.
HRP-konjugierte Sekundärantikörper (anti-mouse-IgG, KPL) werden mit Puffer (LowCross, Candor) auf eine Konzentration von 1 µg/mL (1:1000) eingestellt. 100 µL/Vertiefung dieser Lösung werden in die Mikrotiterplatte pipettiert und inkubiert z.B. 60 min bei 37° C. Danach wird die Mikrotiterplatte gewaschen, z.B. dreimal mit je 200 µL/Vertiefung Waschpuffer (Washing buffer Tris, Candor) für je 5 min.
Den Vertiefungen wird jeweils 100 µL/Vertiefung TMB Substrat hinzugegeben und im Dunkeln 5 min inkubiert. Anschließend wird als Stopplösung 100 µL/Vertiefung H₂SO₄ hinzugefügt und die Extinktion (optische Dichte, OD) der Proben bei 450 nm gemessen.

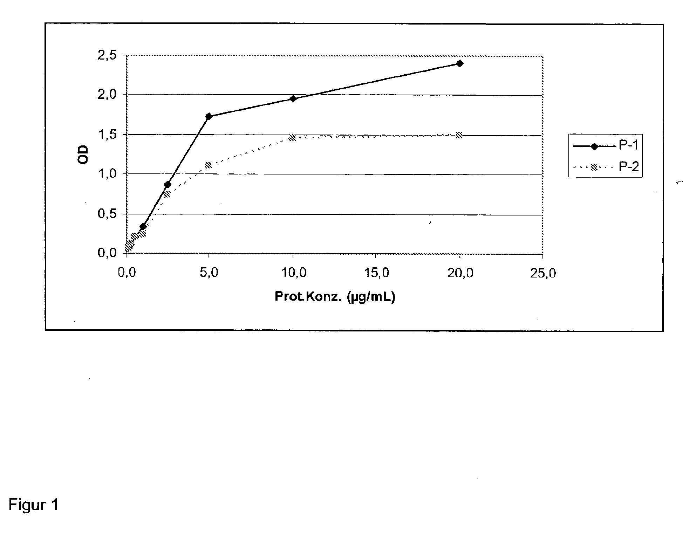

Figur 1 zeigt eine Auswertung der OD-Werte bei Proteinkonzentrationen von 0,1 µg/mL, 0,25 µg/mL, 0,5 µg/mL, 1,0 µg/mL, 2,5 µg/mL, 5,0 µg/mL, 10,0 µg/mL und 20 µg/mL an Protamin-1 und Protamin-2.

### 2.2. ELISA-Verfahren (kompetitives Verfahren)

Alternativ wird das Verfahren in einem ELISA nach einem kompetitiven Verfahren durchgeführt.
Das Ausgangsmaterial ist die aufbereitete fragliche Probe gemäß Ausführungsbeispiel 1, d.h. die Suspension mit dekondensierten Spermien mit einer eingestellten Proteinkonzentration von z.B. 0,1 µg/mL, 0,25 µg/mL, 0,5 µg/mL, 1,0 µg/mL, 2,5 µg/mL, 5,0 µg/mL, 10,0 µg/mL und/oder 20 µg/mL.

Hierzu werden die Proteine Protamin-1 und Protamin-2 z.B. als rekombinante Proteine mit definierter Konzentration, z.B. von 0,1 µg/mL, 0,25 µg/mL, 0,5 µg/mL, 1,0 µg/mL, 2,5 µg/mL, 5,0 µg/mL, 10,0 µg/mL und/oder 20 µg/mL voneinander getrennt auf die Oberfläche einer Mikrotiterplatte gebunden und inkubiert.
Die Mikrotiterplatte wird ausgeklopft und unspezifische Bindungen werden abgesättigt, z.B. während einer Inkubation von 30 min bei 37° C mit 300 µL/Vertiefung Blockierungslösung (Blocking solution, Candor). Danach wird die Mikrotiterplatte gewaschen, z.B. mit 200 µL/Vertiefung Waschpuffer (Washing buffer Tris, Candor) für je 10 sec.
Der Probenextrakt wird mit einer geeigneten Menge monoklonaler anti-Protamin-IgG-Antikörper gemischt und inkubiert, z.B. bei +4° C, wobei 2 separate Ansätze für die Proteine Protamin-1 und Protamin-2 vorgesehen sind.
Zusätzlich kann eine Standardreihe mit definierter Konzentration an Protamin-1 und Protamin-2 mitgeführt werden. Dazu werden die beiden rekombinanten Proteine (P-1 und P-2) auf mindestens drei verschiedene Konzentrationen eingestellt und mit den entsprechenden anti-Protamin-Antikörpern versetzt, d.h. der Ansatz Protamin-1 wird mit anti-Protamin-1-Antikörper versetzt und der Ansatz Protamin-2 wird mit anti-Protamin-2-Antikörper versetzt.
100 µL der Probenextrakt-Antikörper-Mischung pro Vertiefung werden pipettiert und inkubiert, z.B. 90 min bei 37° C. Danach wird die Mikrotiterplatte gewaschen, z.B. dreimal mit je 200 µL/Vertiefung Waschpuffer (Washing buffer Tris, Candor) für je 10 sec.
Die HRP-konjugierten Sekundärantikörper (anti-mouse-IgG, KPL) werden mit Puffer (LowCross, Candor) auf eine definierte Konzentration (z.B. 1 µg/mL) eingestellt. 100 µL/Vertiefung werden pipettiert und 60 min bei 37° C inkubiert. Danach wird die Mikrotiterplatte gewaschen, z.B. dreimal mit je 200 µL/Vertiefung Waschpuffer (Washing buffer Tris, Candor) für je 5 min.
100 µL/Vertiefung TMB Substrat wird pipettiert und im Dunkeln inkubiert. 100 µL/Vertiefung Stopplösung H₂SO₄ wird hinzu pipettiert und die Probenextinktion auf der Mikrotiterplatte bei 450 nm gemessen.
Bei diesem kompetitiven ELISA ist die Intensität des Signals umgekehrt proportional zur Menge des Antigens (Protamin-1 bzw. Protamin-2) der Probe. Das heißt, je mehr freies Protamin im Probenextrakt vorhanden ist, desto weniger Antikörper binden an dem auf der Mikrotiterplatte gebundenen rekombinanten Protamin. Anhand der mitlaufenden Standardreihen von Protamin-1 und Protamin-2 wird die genaue Konzentration von Protamin-1 und Protamin-2 für die jeweilige fragliche Probe kalkuliert. Hieraus errechnet sich dann die Protamin-Protein-Ratio und es kann eine Aussage über die potentielle männliche Fertilität und den Erfolg einer in-vitro-Fertilisation gemacht werden.

### 2.3. Schnelltest

Das erfindungsgemäße Verfahren zur Bestimmung der Fertilität von Spermien, bevorzugt von humanen Spermien wird alternativ mit einem Schnelltest auf einem Teststreifen durchgeführt. Die Durchführung des Schnelltestes ist für jedermann ohne spezielle Kenntnisse und ohne eine aufwendige Laborausstattung an jedem beliebigen Ort möglich. Das Ergebnis, ob Fertilität vorliegt, ist einfach und schnell auswertbar.
Mit diesem Schnelltest werden erstmals die zu detektierenden Proteine Protamin-1 und Protamin-2 jeweils separat qualitativ und semi-quantitativ erfasst. Die Auswertung beinhaltet die Darstellung des Mengenverhältnisses beider Proteine Protamin-1 und Protamin-2 zueinander in einfacher visueller Art und Weise, z.B. in einer Farbreaktion in den Feldern 107, die unmittelbar für das menschliche Auge sichtbar ist und direkt als Ergebnis eine Aussage über die potentielle männliche Fertilität macht.

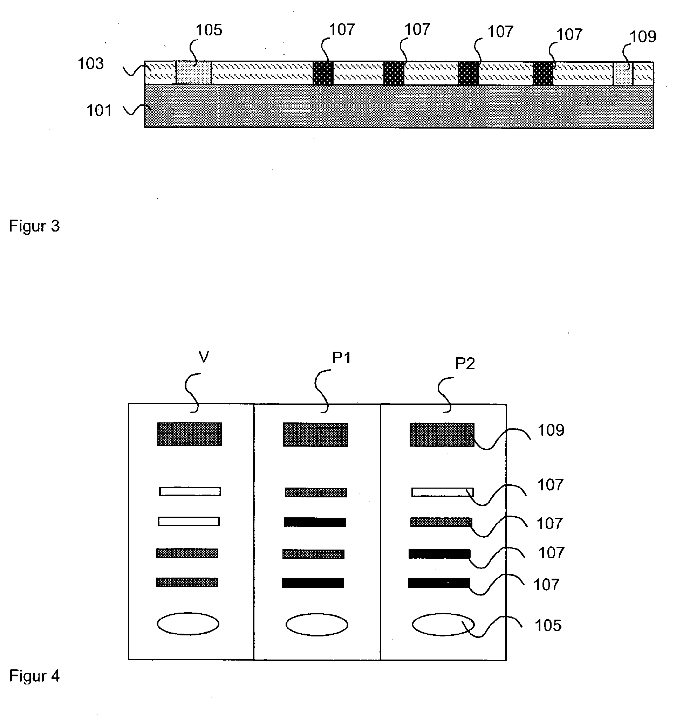

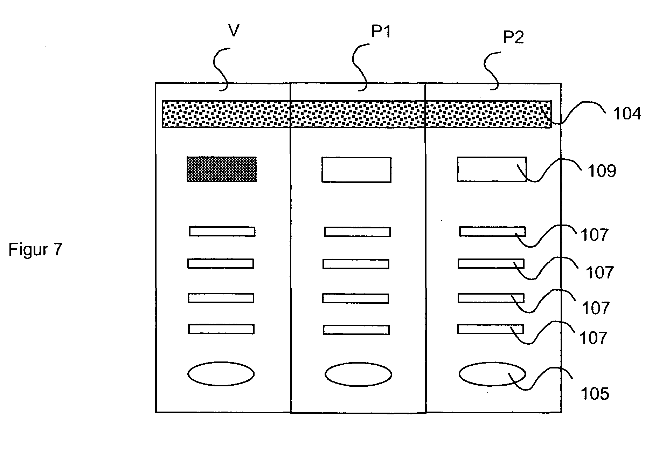

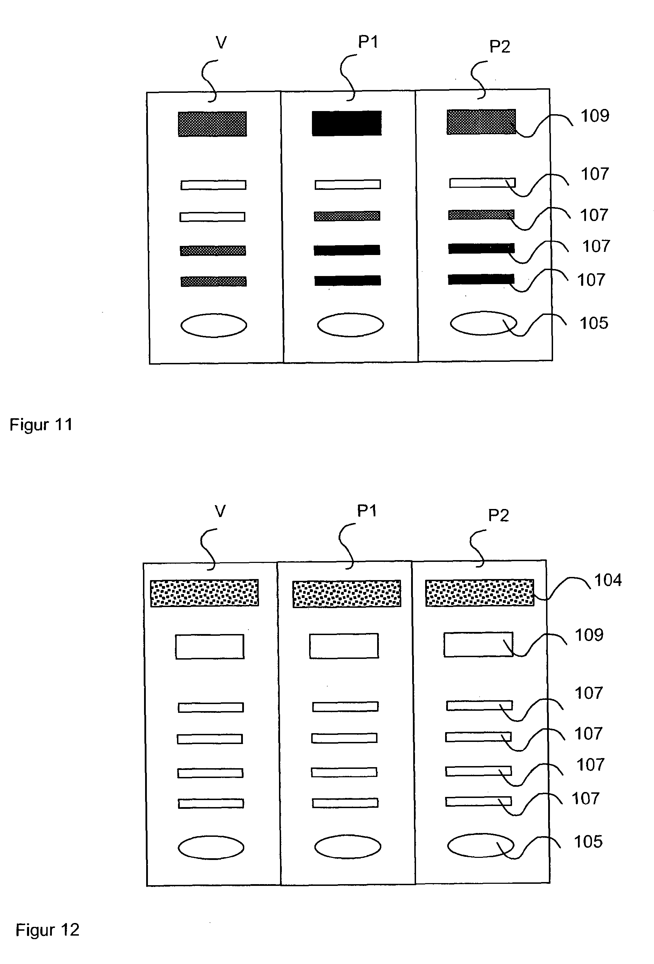

Vorzugsweise weist der erfindungsgemäße Teststreifen eine Grundfläche 101 aus einem geeigneten Material, wie z.B. Nylon auf. Die Grundfläche 101 ist quadratisch oder rund, vorzugsweise rechteckig.
Der erfindungsgemäße Teststreifen weist eine bevorzugte Länge von mindestens 4 cm auf und eine Breite pro Zone von 3 bis 5 mm, vorzugsweise 4 mm.
Weiterhin ist die Grundfläche 101 mit einem geeigneten Material beschichtet, z. B. Celluloseacetat oder Cellulosenitrat, sodass sich eine Auftragsschicht 103 ergibt, die eine ebene Laufoberfläche bildet (siehe Figur 3) und wenige µm dick ist. Der Teststreifen weist mehrere Zonen auf, insbesondere eine Zone V als Spermienvortest, in dem die für den Schnelltest geeignete Spermienkonzentration ermittelt wird. Weiterhin die Zonen P1 und P2 als Protamin-Schnelltest. Die Zone P1 ist zum spezifischen Nachweis des Antigens Protamin-1 ausgebildet und die Zone P2 zum spezifischen Nachweis des Antigens Protamin-2.
Die Auftragsschicht 103 ist durchgängig in den Zonen V, P1 und P2 ausgestaltet. Jede Zone V, P1 und P2 weist auf der Auftragsschicht 103 Felder auf, nämlich jeweils ein Feld 105 für die Probenauftragung einer fraglichen Probe, jeweils mindestens ein Feld 107 und jeweils ein Feld 109 als Kontrollfeld.
Eine bevorzugte Alternative des Teststreifens weist zusätzlich ein Feld 104 auf, das aus einem saugenden Material, z.B. Silica-Gel besteht, sodass die Diffusion der fraglichen Probe entlang der Felder unterstützt und beschleunigt wird. Das Feld 104 befindet sich am Ende des Teststreifens hinter dem Feld 109, sodass die Diffusion der fraglichen Probe von Feld 105 über das mindestens eine Feld 107 zum Feld 109 unterstützt und beschleunigt wird.
Das Feld 104 ist durchgehend für die Zonen V, P1 und P2 ausgebildet (siehe Figur 7), alternativ sind für jede Zone V, P1 und P2 separate Felder 104 ausgebildet (siehe Figur 12, 13 und 14).

### 2.3.1. Spermienvortest

### Vorrichtung

Der Spermienvortest wird in Zone V des Teststreifens durchgeführt. Es handelt sich um einen semi-quantitativen Vortest zur Bestimmung der Spermienkonzentration in der fraglichen Probe z.B. einer lysierten Ejakulatprobe. Anhand des Ergebnisses des Spermienvortests wird die fragliche Probe entweder unverändert weiter für den Protamin-Schnelltest verwendet oder mit einem entsprechenden Volumen Verdünnungspuffer auf eine definierte Spermienkonzentration eingestellt und dann für den Protamin-Schnelltest verwendet.

Der Spermienvortest enthält in Feld 105 gegen Spermien-Oberflächenantigene gerichtete Antikörper. Bevorzugt enthält Feld 105 eine Mischung aus mit Goldkolloid markierten Antikörpern gegen Spermien-Oberflächenantigene und nichtmarkierten Antikörpern gegen Spermien-Oberflächenantigene.
Sobald die fragliche Probe im Feld 105 aufgetragen wird, werden die darin enthaltenen Spermien von den Antikörpern, die gegen die Spermien-Oberflächenantigene gerichtet sind, gebunden und es entsteht ein Komplex aus Spermien-Oberflächenantigenen und den dagegen gerichteten Antikörpern. Dieser Komplex ist durch die Goldkolloidmarkierung mit dem menschlichen Auge als Farbrektion sichtbar, beispielsweise als Rotfärbung.

Mindestens ein Feld 107, bevorzugt mehrere Felder z.B. zwei bis zehn, besonders vorzugsweise vier Felder 107 sind nacheinander zwischen Feld 105 und Kontrollfeld 109 auf dem Teststreifen angeordnet (siehe Figur 2). Figur 13 zeigt eine alternative Ausführungsform mit drei Feldern 107. Figur 14 zeigt eine weitere alternative Ausführungsform mit fünf Feldern 107.
Die Felder 107 sind in gleichen Abständen angeordnet, alternativ sind die Abstände unterschiedlich. Die Felder 107 enthalten unmarkierte Antikörper gegen die Komplexe aus Spermien-Oberflächenantigenen mit den dagegen gerichteten Antikörpern.

Alternativ enthalten die Felder 107 unterschiedliche Mengen an Antikörpern gegen die Komplexe aus Spermien-Oberflächenantigenen mit den dagegen gerichteten Antikörpern, so dass eine unterschiedliche Nachweisempfindlichkeit entsteht.
Bevorzugt weist das dem Kontrollfeld 109 am nächsten gelegene Feld 107 die höchste Konzentration an Antikörpern gegen die Komplexe aus Spermien-Oberflächenantigenen mit den dagegen gerichteten Antikörpern auf.
Die Menge an Antikörpern gegen die Komplexe aus Spermien-Oberflächenantigenen mit den dagegen gerichteten Antikörpern auf den Feldern 107 verringert sich bis zu dem, dem Kontrollfeld 109 am weitesten entfernte Feld 107.

Das Kontrollfeld 109 auf der Zone V weist nichtmarkierte Antikörper gegen kolloidmarkierte Spermien-Oberflächenantigene auf und stellt eine Kontrolle für die Validität des Tests dar.

### Verfahren in Zone V

Eine nach 1. aufbereitete fragliche Probe, z.B. eine Ejakulatprobe wird in geeignetem Volumen z.B. mindestens 50 µL auf Feld 105 der Zone V aufgebracht. Alternativ wird der Teststreifen so in die fragliche Probe eingetaucht, dass nur das Feld 105 der Zone V vollständig durch die fragliche Probe benetzt wird.
Vorzugsweise wird die fragliche Probe, z.B. Ejakulat vorher inkubiert bis zur Verflüssigung, vorzugsweise 30 min bei Raumtemperatur.
Sind in der fraglichen Probe Spermien vorhanden, binden diese an die auf Feld 105 vorgelegten mit Goldkolloid markierten und nichtmarkierten Antikörper gegen Spermien-Oberflächenantigene, sodass markierte und nichtmarkierte Komplexe aus Spermien-Oberflächenantigenen und den dagegen gerichteten Antikörpern gebildet werden. Die mit Goldkolloid markierten Komplexe bewirken in Feld 105 eine dem menschlichen Auge sichtbare Farbreaktion, insbesondere eine Rotfärbung.
Die fragliche Probe mit den darin gebildeten mit Goldkolloid markierten und nichtmarkierten Komplexen aus Spermien-Oberflächenantigenen und den dagegen gerichteten Antikörpern diffundiert entlang des Teststreifens in Zone V und erreicht nacheinander die Felder 107 und das Kontrollfeld 109.
In den Feldern 107 kommen die gebildeten mit Goldkolloid markierten und nichtmarkierten Komplexe aus Spermien-Oberflächenantigenen und den dagegen gerichteten Antikörpern in Kontakt mit den vorgelegten nichtmarkierten Antikörpern gegen die gebildeten Komplexe und in Kontakt mit den Antikörpern gegen Spermien-Oberflächenantigene. Je nach Spermienkonzentration der Probe wird in den Feldern 107 eine Bindung dieses Antikörpers mit gebildeten Komplexen als Farbreaktion in keinem bis allen Feldern 107 sichtbar (siehe Figur 4 bis 7), so dass anhand der Anzahl der Felder 107 mit Farbreaktion eine Aussage über die Spermienkonzentration in der Probe möglich ist.
Die Ausbildung der Farbreaktionen in unterschiedlich vielen Feldern 107 des Teststreifens in Zone V weist auf die Spermienkonzentration der fraglichen Probe hin.
Zeigen mindestens eines, aber nicht alle der Felder 107 in Zone V eine positive Farbreaktion ist die Spermienkonzentration der fraglichen Probe optimal und liegt zwischen 0,2 Mio. Spermien/mL bis 200 Mio. Spermien/mL. Damit kann die fragliche Probe unverändert im Protamin-Schnelltest auf den Zonen P1 und P2 verwendet werden.
Zeigt keines der Felder 107 in Zone V eine positive Farbreaktion ist die Spermienkonzentration der fraglichen Probe zu gering und die fragliche Probe kann zur Testdurchführung nicht so verwendet werden, sondern muss erst aufkonzentriert werden.
Zeigen alle Felder 107 in Zone V eine positive Farbreaktion ist die Spermienkonzentration der fraglichen Probe zu hoch und eine Verdünnung der fraglichen Probe wird empfohlen, so dass die optimale Spermienkonzentration zwischen 0,2 Mio. Spermien/mL bis 200 Mio. Spermien/mL erreicht wird. Vorzugsweise erfolgt die Verdünnung mit Verdünnungspuffer.
Die Bestimmung der Spermienkonzentration in der fraglichen Probe in Zone V ist vorzugsweise nach 10 min abgeschlossen.
Alternativ wird die Spermienkonzentration in der fraglichen Probe, z.B. einer Ejakulatprobe photometrisch bestimmt und dann ggf. auf die optimale Spermienkonzentration von 0,2 Mio. Spermien/mL bis 200 Mio. Spermien/mL eingestellt.

### Auswertung des Spermienvortests

Die Auswertung des Spermienvortests erfolgt nach folgenden Angaben:
a) Der Spermienvortest kann ausgewertet werden, wenn eine Farbreaktion im Kontrollfeld 109 der Zone V des Spermienvortests deutlich sichtbar ist (siehe Figur 4, 5, 6, 7, 11).
b) Der Spermienvortest kann nicht ausgewertet werden, wenn keine Farbreaktion im Kontrollfeld 109 der Zone V des Spermienvortests sichtbar ist.
c) erscheint keine sichtbare Farbreaktion in einem der Felder 107 in Zone V, ist die Spermienkonzentration für den folgenden Protamin-Schnelltest zu gering.
d) Weist der Spermienvortest in Zone V eine Farbreaktion in allen Feldern 107 auf, wird die fragliche Probe, z.B. Ejakulat mit Verdünnungspuffer versehen und auf eine optimale Spermienkonzentration von 0,2 Mio Spermien/mL bis 200 Mio Spermien/mL eingestellt.
e) Weist der Spermienvortest in Zone V eine Farbreaktion in mindestens einem Feld 107 aber nicht allen Feldern 107 auf, liegt eine optimale Spermienkonzentration von 0,2 Mio Spermien/mL bis 200 Mio Spermien/mL vor und der Protamin-Schnelltest kann durchgeführt werden.

### 2.3.2. Protamin-Schnelltest

### Vorrichtung

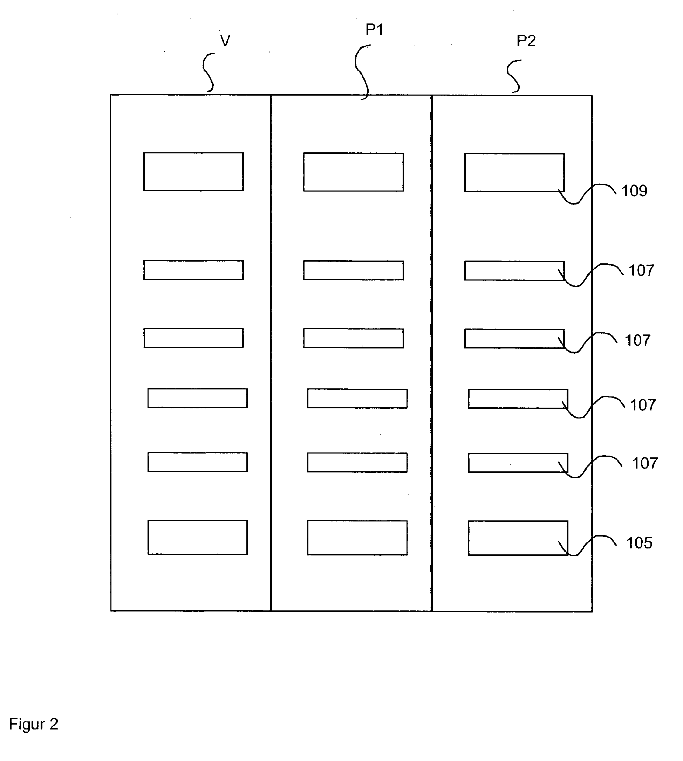

Der Protamin-Schnelltest wird in den Zonen P1 und P2 des Teststreifens durchgeführt (siehe Figur 2, 3). Es handelt sich um einen semi-quantitativen Test zur Bestimmung der Konzentration von Protamin-1 und Protamin-2 in der fraglichen Probe z.B. einer lysierten Ejakulatprobe.
Im Protamin-Schnelltest wird anhand von Farbreaktionen in den Feldern 107 in den Zonen P1 und P2 das Protamin-Protein-Ratio (Verhältnis der Menge von Protamin-1 zu der Menge von Protamin-2) bestimmt. Diese Protamin-Protein-Ratio ist ein valider Prognosemarker für den Erfolg einer nachfolgenden in-vitro-Fertilisation oder intrazytoplasmatischen Spermieninjektion. Liegt die Protamin-Protein-Ratio in Spermien oder Spermatiden einer fraglichen Probe bei 0,8 bis 1,2 Protamin-1 zu 0,8 bis 1,2 Protamin-2, vorzugsweise bei 1 zu 1, dann liegt eine normale Fertilität der Spermien vor und sie können für eine nachfolgende in-vitro-Fertilisation oder intrazytoplasmatische Spermieninjektion verwendet werden. Liegt dagegen ein davon abweichendes Verhältnis vor, so weist dies auf eine verminderte Fertilität bis hin zu Infertilität der Spermien oder Spermatiden in der fraglichen Probe hin. Diese Spermien werden nicht für eine nachfolgende in-vitro-Fertilisation oder intrazytoplasmatische Spermieninjektion verwendet.

### Zone P1

Der Protamin-Schnelltest enthält in der Zone P1 des Teststreifens im Feld 105 anti-Protamin-1-Antikörper. Bevorzugt enthält Feld 105 eine Mischung aus mit Goldkolloid markierten anti-Protamin-1-Antikörper und nichtmarkierten anti-Protamin-1-Antikörpern.
Sobald die fragliche Probe im Feld 105 aufgetragen wird, wird darin enthaltenes Protamin-1 von den anti-Protamin-1-Antikörpern gebunden und es entsteht ein Komplex aus Protamin-1 und anti-Protamin-1-Antikörpern. Dieser Komplex ist durch die Goldkolloidmarkierung mit dem menschlichen Auge als Farbrektion sichtbar, beispielsweise als Rotfärbung.

Mindestens ein Feld 107, bevorzugt mehrere Felder z.B. zwei bis zehn, besonders vorzugsweise vier Felder 107 sind nacheinander zwischen Feld 105 und Kontrollfeld 109 auf dem Teststreifen angeordnet (siehe Figur 2, 3).
Die Felder 107 sind in gleichen Abständen angeordnet, alternativ sind die Abstände unterschiedlich. Die Felder 107 enthalten unmarkierte Antikörper gegen die goldkolloidmarkierten Komplexe aus Protamin-1 und anti-Protamin-1-Antikörper.

Bevorzugt weist das dem Kontrollfeld 109 am nächsten gelegene Feld 107 die höchste Konzentration an Antikörpern gegen die goldkolloidmarkierten Komplexe aus Protamin-1 und anti-Protamin-1-Antikörper auf.
Die Menge an Antikörpern gegen die goldkolloidmarkierten Komplexe aus Protamin-1 und anti-Protamin-1-Antikörper auf den Feldern 107 verringert sich bis zu dem, dem Kontrollfeld 109 am weitesten entfernte Feld 107.

Vorzugsweise entspricht die Anzahl der Felder 107 auf Zone P1 der Anzahl der Felder 107 auf Zone P2.
Der Teststreifen weist in Zone P1 ein Kontrollfeld 109 auf, welches unmarkierte Antikörper gegen goldkolloidmarkierte anti-Protamin-1-Antikörper aufweist und stellt eine Kontrolle für die Validität des Tests dar.

### Zone P2

Der Protamin-Schnelltest enthält in der Zone P2 des Teststreifens im Feld 105 anti-Protamin-2-Antikörper. Bevorzugt enthält Feld 105 eine Mischung aus mit goldkolloidmarkierten anti-Protamin-2-Antikörper und nichtmarkierten anti-Protamin-2-Antikörpern.
Sobald die fragliche Probe im Feld 105 aufgetragen wird, wird darin enthaltenes Protamin-2 von den anti-Protamin-2-Antikörpern gebunden und es entsteht ein Komplex aus Protamin-2 und anti-Protamin-2-Antikörper. Dieser Komplex ist durch die Goldkolloidmarkierung mit dem menschlichen Auge als Farbrektion sichtbar, beispielsweise als Rotfärbung.

Mindestens ein Feld 107, bevorzugt mehrere Felder z.B. zwei bis zehn, besonders vorzugsweise vier Felder 107 sind nacheinander zwischen Feld 105 und Kontrollfeld 109 auf dem Teststreifen angeordnet (siehe Figur 2, 3).
Die Felder 107 sind in gleichen Abständen angeordnet, alternativ sind die Abstände unterschiedlich. Die Felder 107 enthalten unmarkierte Antikörper gegen die goldkolloidmarkierten Komplexe aus Protamin-2 und anti-Protamin-2-Antikörper.

Bevorzugt weist das dem Kontrollfeld 109 am nächsten gelegene Feld 107 die höchste Konzentration an goldkolloidmarkierten Antikörpern gegen die Komplexe aus Protamin-2 und anti-Protamin-2-Antikörper auf.
Die Menge an Antikörpern gegen die goldkolloidmarkierten Komplexe aus Protamin-2 und anti-Protamin-2-Antikörper auf den Feldern 107 verringert sich bis zu dem, dem Kontrollfeld 109 am weitesten entfernte Feld 107.

Vorzugsweise entspricht die Anzahl der Felder 107 auf Zone P2 der Anzahl der Felder 107 auf Zone P1.

Der Teststreifen weist in Zone P2 ein Kontrollfeld 109 auf, welches unmarkierte Antikörper gegen goldkolloidmarkierte anti-Protamin-2-Antikörper aufweist und stellt eine Kontrolle für die Validität des Tests dar.

### Verfahren P1

Die auf die optimale Spermienkonzentration von 0,2 Mio Spermien/mL bis 200 Mio Spermien/mL eingestellte fragliche Probe, z.B. eine Ejakulatprobe wird in geeignetem Volumen z.B. mindestens 50 µL auf Feld 105 in Zone P1 des Teststreifens des Protamin-Schnelltests aufgebracht.
Alternativ wird der Teststreifen so in die fragliche Probe eingetaucht, dass nur das Feld 105 der Zone P1 vollständig durch die fragliche Probe benetzt wird.
In einer weiteren Alternative wird der Teststreifen so in die fragliche Probe eingetaucht, dass nur das Feld 105 der Zone P1 und das Feld 105 der Zone P2 gleichzeitig und vollständig durch die fragliche Probe benetzt werden.

Ist in der fraglichen Probe Protamin-1 vorhanden, bindet dieses an auf Feld 105 vorgelegten goldkolloidmarkierten und nichtmarkierten Antikörpern gegen Protamin-1 (anti-Protamin-1-Antikörper), so dass markierte und nichtmarkierte Komplexe aus Protamin-1 und anti-Protamin-1-Antikörpern gebildet werden.
Die mit Goldkolloid markierten Komplexe bewirken in Feld 105 eine dem menschlichen Auge sichtbare Farbreaktion, insbesondere eine Rotfärbung.

Die fragliche Probe mit den darin gebildeten markierten und nichtmarkierten Komplexen aus Protamin-1 und anti-Protamin-1-Antikörpern diffundiert entlang des Teststreifens in Zone P1 und erreicht nacheinander die Felder 107 und das Kontrollfeld 109.
In den Feldern 107 kommen die gebildeten markierten und nichtmarkierten Komplexe aus Protamin-1 und anti-Protamin-1-Antikörpern in Kontakt mit den vorgelegten nichtmarkierten Antikörpern gegen die gebildeten Komplexe. Je nach Protamin-1-Konzentration in der Probe wird in den Feldern 107 eine Bindung dieses Antikörpers mit gebildeten Komplexen als Farbreaktion in keinem bis allen Feldern 107 sichtbar (siehe Figur 4, 5, 6, 7, 11), so dass anhand der Anzahl der Felder 107 mit Farbreaktion eine Aussage über die Protamin-1-Konzentration in der Probe möglich ist.
Die Ausbildung der Farbreaktionen in unterschiedlich vielen Feldern 107 des Teststreifens in Zone P1 weist auf die Protamin-1-Konzentration der fraglichen Probe hin.

Das Kontrollfeld 109 der Zone P1 weist einen nichtmarkierten Antikörper auf, der gegen goldkolloidmarkierten anti-Protamin-1-Antikörper gerichtet ist. Die Markierung verursacht eine für das menschliche Auge sichtbare Farbreaktion bei Bindung. Diese Farbreaktion dient als Kontrolle, dass der benutzte Teststreifen intakt ist, richtig angewendet wurde und die Probe bis zum Ende des Teststreifens durchgelaufen ist.

Die Bestimmung der Protamin-1-Konzentration in der fraglichen Probe in Zone P1 ist vorzugsweise nach 10 min abgeschlossen.

### Verfahren P2

Die auf die optimale Spermienkonzentration von 0,2 Mio Spermien/mL bis 200 Mio Spermien/mL eingestellte fragliche Probe, z.B. eine Ejakulatprobe wird in geeignetem Volumen z.B. mindestens 50 µL auf Feld 105 in Zone P2 des Teststreifens des Protamin-Schnelltests aufgebracht.
Alternativ wird der Teststreifen so in die fragliche Probe eingetaucht, dass nur das Feld 105 in Zone P2 vollständig durch die fragliche Probe benetzt wird.
In einer weiteren Alternative wird der Teststreifen so in die fragliche Probe eingetaucht, dass nur das Feld 105 der Zone P1 und das Feld 105 der Zone P2 gleichzeitig und vollständig durch die fragliche Probe benetzt werden.

Ist in der fraglichen Probe Protamin-2 vorhanden, bindet dieses an auf Feld 105 vorgelegten goldkolloidmarkierten und nichtmarkierten Antikörpern gegen Protamin-2 (anti-Protamin-2-Antikörper), sodass markierte und nichtmarkierte Komplexe aus Protamin-2 und anti-Protamin-2-Antikörpern gebildet werden.
Die mit Goldkolloid markierten Komplexe bewirken in Feld 105 eine dem menschlichen Auge sichtbare Farbreaktion, insbesondere eine Rotfärbung.

Die fragliche Probe mit den darin gebildeten markierten und nichtmarkierten Komplexen aus Protamin-2 und anti-Protamin-2-Antikörpern diffundiert entlang des Teststreifens in Zone P2 und erreicht nacheinander die Felder 107 und das Kontrollfeld 109.

In den Feldern 107 kommen die gebildeten markierten und nichtmarkierten Komplexe aus Protamin-2 und anti-Protamin-2-Antikörpern in Kontakt mit den vorgelegten nichtmarkierten Antikörpern gegen die gebildeten Komplexe. Je nach Protamin-2-Konzentration in der Probe wird in den Feldern 107 eine Bindung dieses Antikörpers mit gebildeten Komplexen als Farbreaktion in keinem bis allen Feldern 107 sichtbar (siehe Figur 4, 5, 6, 7, 11), sodass anhand der Anzahl der Felder 107 mit Farbreaktion eine Aussage über die Protamin-2-Konzentration in der Probe möglich ist.
Die Ausbildung der Farbreaktionen in unterschiedlich vielen Feldern 107 des Teststreifens in Zone P2 weist auf die Protamin-2-Konzentration der fraglichen Probe hin.

Das Kontrollfeld 109 der Zone P2 weist einen nichtmarkierten Antikörper auf, der gegen goldkolloidmarkierten anti-Protamin-2-Antikörper gerichtet ist. Die Markierung verursacht eine für das menschliche Auge sichtbare Farbreaktion bei Bindung. Diese Farbreaktion dient als Kontrolle, dass der benutzte Teststreifen intakt ist, richtig angewendet wurde und die Probe bis zum Ende des Teststreifens durchgelaufen ist.

Die Bestimmung der Protamin-2-Konzentration in der fraglichen Probe in Zone P2 ist vorzugsweise nach 10 min abgeschlossen.

### Auswertung des Protamin-Schnelltests

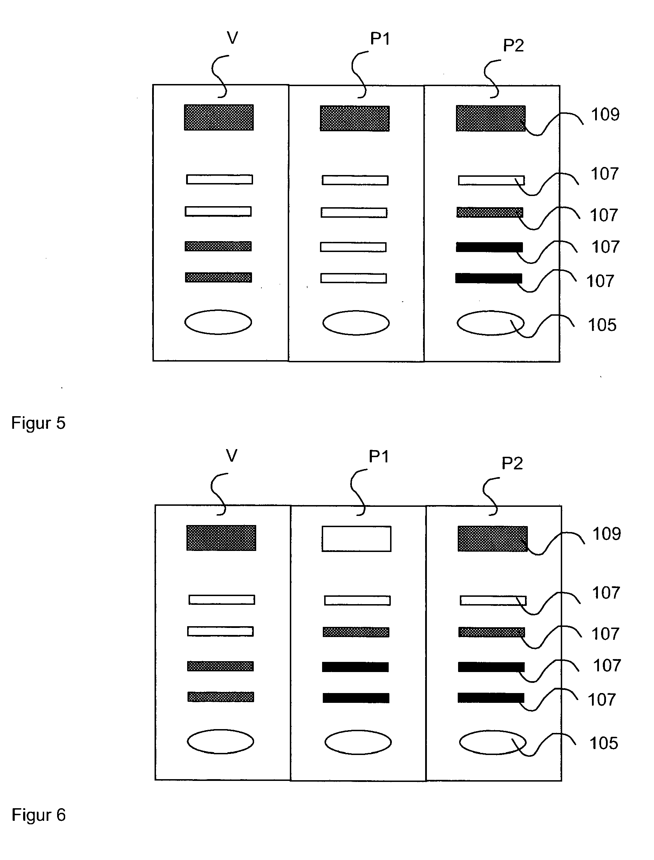

Der Protamin-Schnelltest kann ausgewertet werden, wenn im Kontrollfeld 109 der Zone P1 und im Kontrollfeld 109 der Zone P2 die Farbreaktionen deutlich sichtbar sind (siehe Figur 4, 5, 11).
Der Protamin-Schnelltest kann nicht ausgewertet werden, wenn im Kontrollfeld 109 der Zone P1 oder im Kontrollfeld 109 der Zone P2 eine oder beide Farbreaktionen nicht deutlich sichtbar sind (s. Figur 6, 7).

### 2.3.3. Auswertung des Schnelltests

Die Spermien in der fraglichen Probe sind fertil, wenn folgende Bedingungen erfüllt sind:
a) die Anzahl der Felder 107 in Zone P1, die eine Farbreaktion aufweisen, muss der Anzahl der Felder 107 in Zone P2, die eine Farbreaktion aufweisen, entsprechen,
   und
b) die Position der Felder 107 in Zone P1, die eine Farbreaktion aufweisen, muss der Position der Felder 107 in Zone P2, die eine Farbreaktion aufweisen, entsprechen,
sodass ein Protamin-Protein-Ratio von 0,8 bis 1,2 Protamin-1 zu 0,8 bis 1,2 Protamin-2 vorliegt, vorzugsweise ein Protamin-Protein-Ratio von 1 zu 1.

Ein Beispiel für eine fertile fragliche Probe zeigt Figur 11. Die Probe zeigt nach Durchführung des Verfahrens drei Felder 107 in Zone P1, die eine Farbreaktion aufweisen und drei Felder 107 in Zone P2, die ebenfalls eine Farbreaktion aufweisen, wobei die jeweils drei Felder 107 in Zone P1, die eine Farbreaktion aufweisen sowie die drei Felder 107 in Zone P2, die eine Farbreaktion aufweisen, an gleicher Position bezüglich Feld 105 und Feld 109 gelegen sind. Damit liegt in diesem Beispiel der Figur 11 als Testergebnis eine Protamin-Protein-Ratio (Verhältnis der Menge von Protamin-1 zu der Menge von Protamin-2) von 1 zu 1 vor.

Die Spermien in der fraglichen Probe sind infertil, wenn folgende Bedingungen erfüllt sind:
a) die Anzahl der Felder 107 in Zone P1 mit Farbreaktion entspricht nicht der Anzahl der Felder 107 in Zone P2 mit Farbreaktion,
   oder
b) die Position der Felder 107 in Zone P1 mit Farbreaktion entspricht nicht der Position der Felder 107 in Zone P2 mit Farbreaktion,
so dass keine Protamin-Protein-Ratio von 0,8 bis 1,2 Protamin-1 zu 0,8 bis 1,2 Protamin-2 vorliegt, vorzugsweise kein Protamin-Protein-Ratio von 1 zu 1.

Ein Beispiel für eine infertile fragliche Probe zeigt Figur 4. Die Probe zeigt nach Durchführung des Verfahrens vier Felder 107 in Zone P1, die eine Farbreaktion aufweisen und drei Felder 107 in Zone P2, die ebenfalls eine Farbreaktion aufweisen.

Ein weiteres Beispiel für eine infertile fragliche Probe zeigt Figur 5. Die Probe zeigt nach Durchführung des Verfahrens kein Feld 107 in Zone P1, das eine Farbreaktion aufweist und drei Felder 107 in Zone P2, die eine Farbreaktion aufweisen.

### Abbildungslegenden

Figur 1 zeigt die grafische Darstellung der ermittelten optischen Dichten (OD-Werte) bei Proteinkonzentrationen von 0,1 µg/mL, 0,25 µg/mL, 0,5 µg/mL, 1,0 µg/mL, 2,5 µg/mL, 5,0 µg/mL, 10,0 µg/mL und 20 µg/mL an Protamin-1 ( 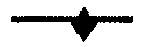 P-1) und Protamin-2 (---□--- P2).
Figur 2 zeigt beispielhaft eine Aufsicht einer möglichen Anordnung der Zonen V, P1 und P2 und der Felder 105, 107 und 109 auf einem rechteckigen Teststreifen. Die Zonen sind parallel nebeneinander angeordnet und Zone P1 befindet sich räumlich zwischen den Zonen P2 und V.
   Alternativ befindet sich Zone P2 räumlich zwischen den Zonen P1 und V.
   Alternativ befindet sich Zone V räumlich zwischen den Zonen P1 und P2.
   Alternativ befinden sich isolierende Trennabschnitte zwischen den Zonen V, P1 und P2.
Figur 3 zeigt einen Querschnitt zu einer Anordnung der erfindungsgemäßen Vorrichtung gemäß Figur 2, insbesondere die Grundfläche des Teststreifens 101, die Auftragsschicht 103, sowie die Felder 105, 107 und 109 in einer Zone.
Figur 4 zeigt die Zonen V, P1 und P2 des Teststreifen nach Durchführung des Verfahrens zum Spermienvortest und zum Protamin-Schnelltest. Die Kontrollfelder 109 in den Zonen V, P1 und P2 zeigen jeweils eine Farbreaktion. Damit liegt ein valides Testergebnis vor. In Zone V weisen zwei Felder 107 eine Farbreaktion auf. Dies zeigt an, dass die Spermienkonzentration in der fraglichen Probe innerhalb der für die Durchführung des Tests optimalen Spermienkonzentration von 0,2 Mio. Spermien/mL bis 200 Mio. Spermien/mL liegt. In der Zone P1 weisen vier Felder 107 eine Farbreaktion auf. In der Zone P2 weisen drei Felder 107 eine Farbreaktion auf. Dies zeigt an, dass keine Protamin-Protein-Ratio von 0,8 bis 1,2 Protamin-1 zu 0,8 bis 1,2 Protamin-2 vorliegt, vorzugsweise keine Protamin-Protein-Ratio von 1 zu 1. Damit sind die Spermien in der fraglichen Probe infertil.
Figur 5 zeigt die Zonen V, P1 und P2 des Teststreifens nach Durchführung des Verfahrens zum Spermienvortest und zum Protamin-Schnelltest. Die Kontrollfelder 109 in den Zonen V, P1 und P2 zeigen jeweils eine Farbreaktion. Damit liegt ein valides Testergebnis vor. In Zone V weisen zwei Felder 107 eine Farbreaktion auf. Dies zeigt an, dass die Spermienkonzentration in der fraglichen Probe innerhalb der für die Durchführung des Tests optimalen Spermienkonzentration von 0,2 Mio. Spermien/mL bis 200 Mio. Spermien/mL liegt. In der Zone P1 weist kein Feld 107 eine Farbreaktion auf. In der Zone P2 weisen drei Felder 107 eine Farbreaktion auf. Dies zeigt an, dass keine Protamin-Protein-Ratio von 0,8 bis 1,2 Protamin-1 zu 0,8 bis 1,2 Protamin-2 vorliegt, vorzugsweise keine Protamin-Protein-Ratio von 1 zu 1. Damit sind die Spermien in der fraglichen Probe infertil.
Figur 6 zeigt die Zonen V, P1 und P2 des Teststreifens nach Durchführung des Verfahrens zum Spermienvortest und zum Protamin-Schnelltest. Die Kontrollfelder 109 in den Zonen V und P2 zeigen jeweils eine Farbreaktion. Das Kontrollfeld 109 in der Zone P1 zeigt keine Farbreaktion. Damit liegt kein valides Testergebnis vor, so dass keine Aussage über die Fertilität der Spermien gemacht werden kann.
Figur 7 zeigt die Zonen V, P1 und P2 des Teststreifens nach Durchführung des Verfahrens zum Spermienvortest und zum Protamin-Schnelltest. Nur das Kontrollfeld 109 in Zone V zeigt eine Farbreaktion. Die Kontrollfelder 109 in den Zonen P1 und P2 zeigen keine Farbreaktion. Damit liegt kein valides Testergebnis vor, so dass keine Aussage über die Fertilität der Spermien gemacht werden kann. Dargestellt ist ein Teststreifen mit einem durchgehenden Feld 104.

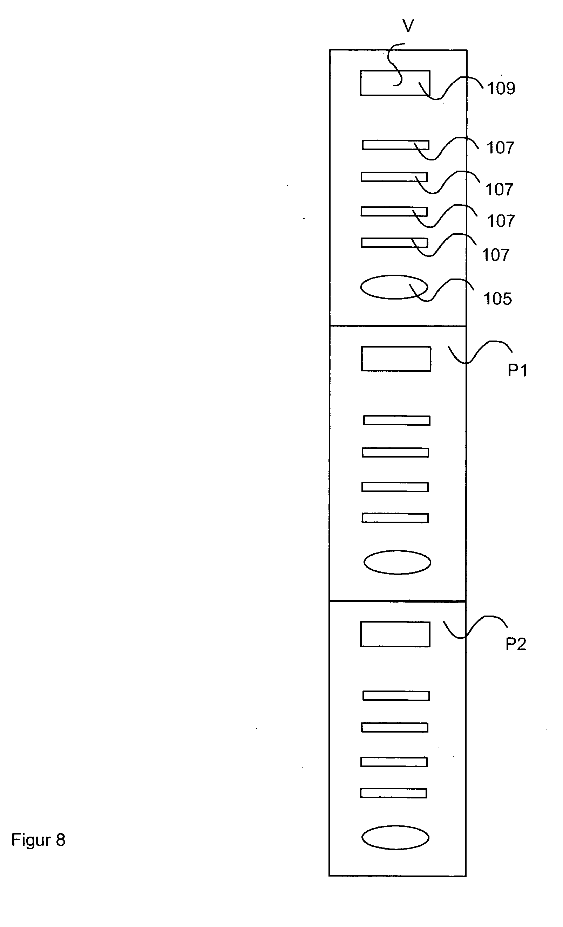

Figur 8 zeigt eine Alternative einer möglichen Anordnung der Zonen V, P1 und P2 auf einem rechteckigen Teststreifen. Die Zonen sind nacheinander angeordnet und Zone P1 befindet sich räumlich zwischen den Zonen P2 und V.
   Alternativ befindet sich Zone P2 räumlich zwischen den Zonen P1 und V.
   Alternativ befindet sich Zone V räumlich zwischen den Zonen P1 und P2.

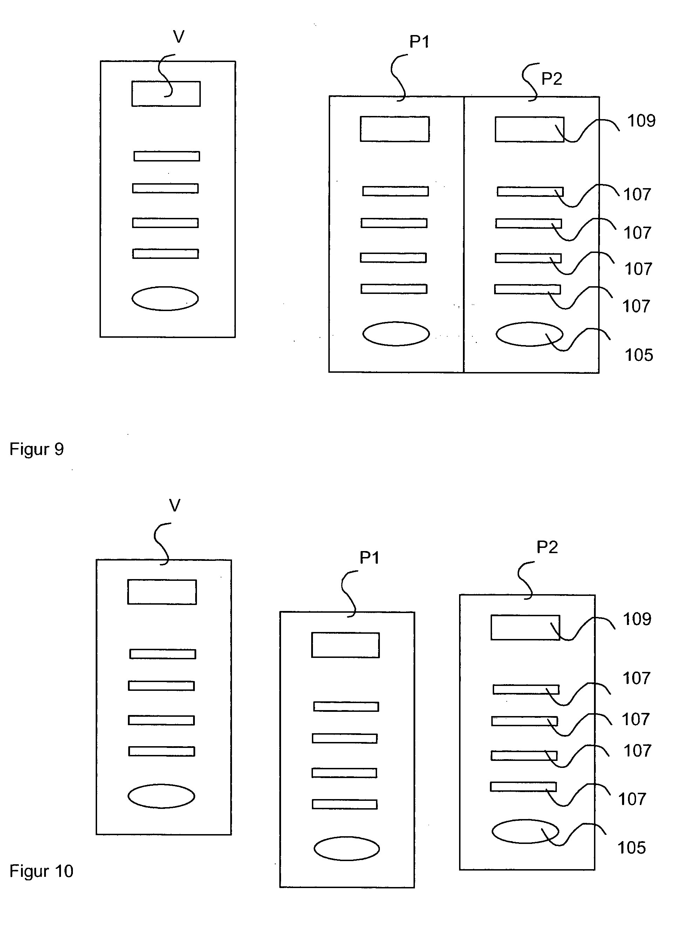

Figur 9 zeigt eine weitere Alternative einer möglichen Anordnung der Zonen V, P1 und P2. Dabei befindet sich Zone V auf einem separaten Teststreifen und die Zonen P1 und P2 gemeinsam auf einem Teststreifen.
Figur 10 zeigt eine weitere Alternative einer möglichen Anordnung der Zonen V, P1 und P2. Dabei befinden sich die Zonen V, P1 und P2 jeweils auf separaten Teststreifen.
Fig. 11 zeigt die Zonen V, P1 und P2 des Teststreifens nach Durchführung des Verfahrens zum Spermienvortest und zum Protamin-Schnelltest. Die Kontrollfelder 109 in den Zonen V, P1 und P2 zeigen jeweils eine Farbreaktion. Damit liegt ein valides Testergebnis vor. In Zone V weisen zwei Felder 107 eine Farbreaktion auf. Dies zeigt an, dass die Spermienkonzentration in der fraglichen Probe innerhalb der für die Durchführung des Tests optimalen Spermienkonzentration von 0,2 Mio. Spermien/mL bis 200 Mio. Spermien/mL liegt. In der Zone P1 weisen drei Felder 107 eine Farbreaktion auf. In der Zone P2 weisen ebenfalls drei Felder 107 eine Farbreaktion auf. Dies zeigt an, dass eine Protamin-Protein-Ratio von 0,8 bis 1,2 Protamin-1 zu 0,8 bis 1,2 Protamin-2 vorliegt, vorzugsweise eine Protamin-Protein-Ratio von 1 zu 1. Damit sind die Spermien in der fraglichen Probe fertil.
Figur 12 zeigt die Zonen V, P1 und P2 des Teststreifens. Dargestellt ist ein Teststreifen mit für jede Zone (V, P1 und P2) separaten Feldern 104. Jede Zone (V, P1 und P2) weist vier Felder 107 auf.

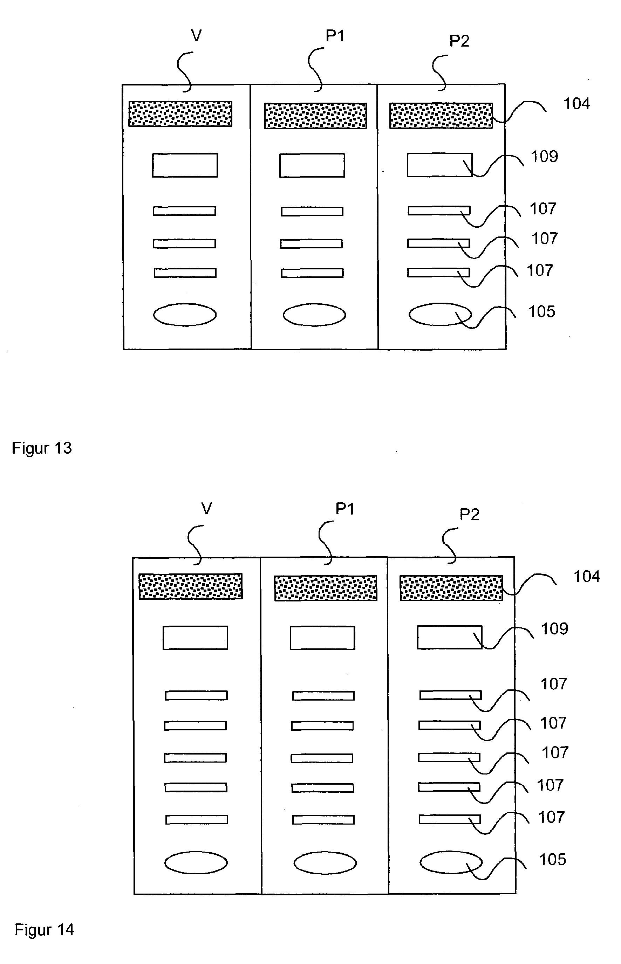

Figur 13 zeigt die Zonen V, P1 und P2 des Teststreifens. Dargestellt ist ein Teststreifen mit für jede Zone (V, P1 und P2) separaten Feldern 104. Jede Zone (V, P1 und P2) weist drei Felder 107 auf.
Figur 14 zeigt die Zonen V, P1 und P2 des Teststreifens. Dargestellt ist ein Teststreifen mit für jede Zone (V, P1 und P2) separaten Feldern 104. Jede Zone (V, P1 und P2) weist fünf Felder 107 auf.

### Bezugszeichenliste

- V: Zone V für den Spermienvortest
- P1: Zone P1 zum Nachweis für Protamin-1
- P2: Zone P2 zum Nachweis für Protamin-2
- 101: Grundfläche des Teststreifens
- 103: Auftragsschicht
- 104: Saugfeld
- 105: Feld für Probenauftragung
- 107: Feld für Reaktion Antigen mit Antikörper
- 109: Kontrollfeld

## Patentansprüche

1. Immunologisches Verfahren zur Bestimmung der Fertilität männlicher Säugetiere und Menschen, wobei die Konzentration an Protamin-1 und Protamin-2 und das Verhältnis zwischen Protamin-1 und Protamin-2, die Protamin-Protein-Ratio, in einer fraglichen Probe eines zu untersuchenden Individuums *in vitro* ermittelt wird, **dadurch gekennzeichnet, dass** die Konzentration an Protamin-1 und Protamin-2 und die Protamin-Protein-Ratio mittels Schnelltest auf einem Teststreifen ermittelt wird, wobei der Schnelltest auf dem Teststreifen einen Spermienvortest und einen Protamin-Schnelltest umfasst.

2. Verfahren nach Anspruch 1 **dadurch gekennzeichnet, dass** die fragliche Probe eines zu untersuchenden Individuums Ejakulat, Hodengewebe oder Nebenhodengewebe ist.

3. Verfahren nach einem der vorangehenden Ansprüche **dadurch gekennzeichnet, dass** im immunologischen Verfahren ein für Protamin-1 spezifischer Antikörper (anti-Protamin-1-Antikörper) und ein für Protamin-2 spezifischer Antikörper (anti-Protamin-2-Antikörper) eingesetzt wird, wobei die Bindung von Protamin-1 an den anti-Protamin-1-Antikörper und die Bindung von Protamin-2 an den anti-Protamin-2-Antikörper erfolgt und diese Bindungen durch Farbreaktion sichtbar gemacht werden.

4. Verfahren nach Anspruch 1 bis 3 **dadurch gekennzeichnet, dass** im Spermienvortest die Spermienkonzentration ermittelt wird, wobei Antikörper gegen Spermien-Oberflächenantigene verwendet werden und wobei die Bindungen dieser Antikörper an Spermien-Oberflächenantigene durch Farbreaktion sichtbar gemacht wird.

5. Verfahren nach Anspruch 1 bis 4 **dadurch gekennzeichnet, dass** im Protamin-Schnelltest die Konzentration an Protamin-1 und Protamin-2 ermittelt wird, wobei die Konzentration an Protamin-1 mit anti-Protamin-1-Antikörpern ermittelt wird und die Konzentration an Protamin-2 mit anti-Protamin-2-Antikörpern, wobei diese Bindungen durch Farbreaktion sichtbar gemacht werden.

6. Verfahren nach Anspruch 1 bis 5 **dadurch gekennzeichnet, dass** die im Spermienvortest eingesetzten Antikörper gegen Spermien-Oberflächenantigene eine Mischung aus mit Goldkolloid markierten Antikörpern mit nichtmarkierten Antikörpern ist.

7. Verfahren nach Anspruch 1 bis 5 **dadurch gekennzeichnet, dass** die im Protamin-Schnelltest eingesetzten anti-Protamin-1-Antikörper und die anti-Protamin-2-Antikörper eine Mischung aus mit Goldkolloid markierten Antikörpern mit nichtmarkierten Antikörpern sind.

8. Teststreifen zur Durchführung eines immunologischen Verfahrens gemäß der Ansprüche 1 bis 7 **dadurch gekennzeichnet, dass** er eine Grundfläche (101) und eine Auftragschicht (103) umfasst, wobei auf der Auftragschicht (103) eine Zone V zur Durchführung des Spermienvortestes zur Ermittlung der Konzentration der Spermien vorhanden ist, ferner eine Zone P1 die einen für Protamin-1 spezifischen Antikörper enthält zur Ermittlung der Konzentration von Protamin-1 und eine Zone P2 die einen für Protamin-2 spezifischen Antikörper enthält zur Ermittlung der Konzentration von Protamin-2.

9. Teststreifen nach Anspruch 8, **dadurch gekennzeichnet, dass** auf den Zonen V, P1 und P2 Felder vorhanden sind, nämlich jeweils ein Feld (105) für die Probenauftragung einer fraglichen Probe, jeweils mindestens ein Feld (107) und jeweils ein Feld (109) als Kontrollfeld.

10. Teststreifen nach Anspruch 8 und 9 **dadurch gekennzeichnet, dass** in Zone V die Durchführung des Spermienvortests zur Ermittlung der Konzentration der Spermien erfolgt, wobei Feld (105) Antikörper gegen Spermien-Oberflächenantigene aufweist und das mindestens eine Feld (107) Antikörper gegen Komplexe aus Spermien-Oberflächenantigen und den dagegen gerichteten Antikörper aufweist, wobei diese Bindungen durch Farbreaktion sichtbar gemacht werden.

11. Teststreifen nach Anspruch 8 bis 10 **dadurch gekennzeichnet, dass** in Zone P1 die Konzentration an Protamin-1 ermittelt wird, wobei Feld (105) anti-Protamin-1-Antikörper aufweist und das mindestens eine Feld (107) Antikörper, die an den Komplex aus Protamin-1 und anti-Protamin-1-Antikörper binden, aufweist, wobei diese Bindungen durch Farbreaktion sichtbar gemacht werden.

12. Teststreifen nach Anspruch 8 bis 10 **dadurch gekennzeichnet, dass** in Zone P2 die Konzentration an Protamin-2 ermittelt wird, wobei Feld (105) anti-Protamin-2-Antikörper aufweist und das mindestens eine Feld (107) Antikörper, die an den Komplex aus Protamin-2 und anti-Protamin-2-Antikörper binden, aufweist, wobei diese Bindungen durch Farbreaktion sichtbar gemacht werden.

13. Teststreifen nach Anspruch 8 bis 12 **dadurch gekennzeichnet, dass** die Zonen V, P1 und P2 parallel nebeneinander angeordnet sind.

14. Verwendung eines Teststreifens nach Anspruch 8 bis 13 zur Ermittlung der Konzentration an Protamin-1 und Protamin-2 und des Verhältnisses zwischen Protamin-1 und Protamin-2 als Protamin-Protein-Ratio in einer fraglichen Probe eines zu untersuchenden Individuums zur Bestimmung der Fertilität der Spermien in vitro.

## Claims

1. Immunological method for determining the fertility of male mammals and humans, in which the concentration of protamine-1 and protamine-2 and the ratio between protamine-1 and protamine-2, the protamine-protein ratio, is determined *in vitro* in a relevant sample from an individual to be tested, **characterised in that** the concentration of protamine-1 and protamine-2 and the protamine-protein ratio is determined by a rapid test on a test strip, the rapid test on the test strip comprising a preliminary sperm test and a rapid protamine test.

2. Method according to claim 1, **characterised in that** the sample in question is ejaculate, testicular tissue or epididymal tissue from an individual to be tested.

3. Method according to one of the preceding claims, **characterised in that** in the immunological method an antibody specific for protamine-1 (anti-protamine-1-antibody) and an antibody specific for protamine-2 (anti-protamine-2-antibody) is used, wherein protamine-1 binds to the anti-protamine-1-antibody and protamine-2 binds to the anti-protamine-2-antibody and these binding reactions are visualised by a staining reaction.

4. Method according to claim 1 to 3, **characterised in that** in the preliminary sperm test the sperm concentration is determined using antibodies against sperm surface antigens and the binding of these antibodies to sperm surface antigens is visualised by a staining reaction.

5. Method according to claim 1 to 4, **characterised in that** in the rapid protamine test the concentration of protamine-1 and protamine-2 is determined, the concentration of protamine-1 being determined using anti-protamine-1-antibodies and the concentration of protamine-2 being determined using anti-protamine-2-antibodies, these binding reactions being visualised by a staining reaction.

6. Method according to claims 1 to 5, **characterised in that** the antibodies against sperm surface antigens used in the preliminary sperm test are a mixture of antibodies labelled with colloidal gold and non-labelled antibodies.

7. Method according to claim 1 to 5, **characterised in that** the anti-protamine-1-antibodies used in the rapid protamine test and the anti-protamine-2-antibodies are a mixture of antibodies labelled with colloidal gold and non-labelled antibodies.

8. Test strip for carrying out an immunological method according to claims 1 to 7, **characterised in that** it comprises a base surface (101) and an applied coating (103), the applied coating (103) having a zone V thereon for carrying out the preliminary sperm test to determine the concentration of sperm, as well as a zone P1 that contains an antibody specific for protamine-1 for determining the concentration of protamine-1 and a zone P2 that contains an antibody specific for protamine-2 for determining the concentration of protamine-2.

9. Test strip according to claim 8, **characterised in that** areas are provided on each of the zones V, P1 and P2, namely an area (105) for the application of a relevant sample, at least one area (107) and an area (109) as a control area.

10. Test strip according to claims 8 and 9, **characterised in that** in zone V the preliminary sperm test is carried out to determine the concentration of sperm, the area (105) containing antibodies against sperm surface antigens and the at least one area (107) containing antibodies against complexes of sperm surface antigen and the antibodies directed against it, these binding reactions being visualised by a staining reaction.

11. Test strip according to claims 8 to 10, **characterised in that** in zone P1 the concentration of protamine-1 is determined, the area (105) comprising anti-protamine-1-antibodies and the at least one area (107) comprising antibodies that bind to the complex of protamine-1 and anti-protamine-1-antibodies, these binding reactions being visualised by a staining reaction.

12. Test strip according to claims 8 to 10, **characterised in that** in zone P2 the concentration of protamine-2 is determined, the area (105) comprising anti-protamine-2-antibodies and the at least one area (107) comprising antibodies that bind to the complex of protamine-2 and anti-protamine-2-antibodies, these binding reactions being visualised by a staining reaction.

13. Test strip according to claims 8 to 12, **characterised in that** the zones V, P1 and P2 are arranged in parallel alongside one another.

14. Use of a test strip according to claims 8 to 13 for determining the concentration of protamine-1 and protamine-2 and the ratio between protamine-1 and protamine-2 as a protamine-protein ratio in a relevant sample from an individual to be tested in order to determine the fertility of the sperm *in vitro.*

## Revendications

1. Procédé immunologique destiné à déterminer la fertilité des mammifères masculins et des hommes, dans lequel la concentration en protamine-1 et en protamine-2 et le rapport entre la protamine-1 et la protamine-2, le ratio protamine-protéine, sont déterminés *in vitro* dans un échantillon mis en cause d'un individu à examiner, **caractérisé en ce que** la concentration en protamine-1 et en protamine-2 et le ratio protamine-protéine sont déterminés au moyen d'un test rapide sur bandelette réactive, le test rapide sur bandelette réactive comprenant un prétest de spermatozoïdes et un test de dépistage rapide de la protamine.

2. Procédé selon la revendication 1, **caractérisé en ce que** l'échantillon mis en cause d'un individu à examiner est un éjaculat, un tissu testiculaire ou un tissu épididymaire.

3. Procédé selon l'une quelconque des revendications précédentes, **caractérisé en ce que**, dans le procédé immunologique, un anticorps spécifique à la protamine-1 (anticorps anti-protamine-1) et un anticorps spécifique à la protamine-2 (anticorps anti-protamine-2) sont utilisés, la liaison de la protamine-1 à l'anticorps anti-protamine-1 et la liaison de la protamine-2 à l'anticorps anti-protamine-2 ayant lieu et ces liaisons étant rendues visibles par une réaction colorée.

4. Procédé selon la revendication 1 à 3, **caractérisé en ce que** la concentration en spermatozoïdes est déterminée lors du prétest de spermatozoïdes, des anticorps dirigés contre des antigènes de surface des spermatozoïdes étant utilisés et les liaisons de ces anticorps à des antigènes de surface des spermatozoïdes étant rendues visibles par une réaction colorée.

5. Procédé selon la revendication 1 à 4, **caractérisé en ce que** la concentration en protamine-1 et en protamine-2 est déterminée dans le test de dépistage rapide de la protamine, la concentration en protamine-1 étant déterminée à l'aide d'anticorps anti-protamine-1 et la concentration en protamine-2 étant déterminée à l'aide d'anticorps anti-protamine-2, ces liaisons étant rendues visibles par une réaction colorée.

6. Procédé selon la revendication 1 à 5, **caractérisé en ce que** les anticorps utilisés dans le prétest de spermatozoïdes et dirigés contre des antigènes de surface des spermatozoïdes est un mélange d'anticorps marqués à l'or colloïdal et d'anticorps non marqués.

7. Procédé selon la revendication 1 à 5, **caractérisé en ce que** les anticorps anti-protamine-1 et les anticorps anti-protamine-2 utilisés dans le test rapide de dépistage de la protamine sont un mélange d'anticorps marqués à l'or colloïdal et d'anticorps non marqués.

8. Bandelette réactive destinée à réaliser un procédé immunologique selon les revendications 1 à 7, **caractérisée en ce qu'**elle comprend une surface de base (101) et une couche déposée (103), une zone V étant présente sur la couche déposée (103) pour la réalisation du prétest de spermatozoïdes destinée à déterminer la concentration en spermatozoïdes, en outre une zone P1 qui contient un anticorps spécifique à la protamine 1 destinée à déterminer la concentration en protamine-1 et une zone P2 qui contient un anticorps spécifique à la protamine 2 destinée à déterminer la concentration en protamine-2.

9. Bandelette réactive selon la revendication 8, **caractérisée en ce que** des champs se trouvent sur les zones V, P1 et P2, à savoir, à chaque fois un champ (105) pour l'application d'un échantillon mis en cause, à chaque fois au moins un champ (107) et à chaque fois un champ (109) comme champ témoin.

10. Bandelette réactive selon la revendication 8 et 9, **caractérisée en ce que** la réalisation du prétest de spermatozoïdes destiné à déterminer la concentration en spermatozoïdes a lieu dans la zone V, le champ (105) présentant des anticorps dirigés contre des antigènes de surface des spermatozoïdes et le au moins un champ (107) présentant des anticorps dirigés contre des complexes d'antigènes de surface des spermatozoïdes et des anticorps dirigés contre ces derniers, ces liaisons étant rendues visibles par une réaction colorée.

11. Bandelette réactive selon la revendication 8 à 10, **caractérisée en ce que** la concentration en protamine-1 est déterminée dans la zone P1, le champ (105) présentant des anticorps anti-protamine-1 et le au moins un champ (107) présentant des anticorps qui se lient au complexe de la protamine-1 et d'anticorps anti-protamine-1, ces liaisons étant rendues visibles par une réaction colorée.

12. Bandelette réactive selon la revendication 8 à 10, **caractérisée en ce que** la concentration en protamine-2 est déterminée dans la zone P2, le champ (105) présentant des anticorps anti-protamine-2 et le au moins un champ (107) présentant des anticorps qui se lient au complexe de la protamine-2 et d'anticorps anti-protamine-2, ces liaisons étant rendues visibles par une réaction colorée.

13. Bandelette réactive selon la revendication 8 à 12, **caractérisée en ce que** les zones V, P1 et P2 sont agencées l'une à côté de l'autre parallèlement.

14. Utilisation d'une bandelette réactive selon la revendication 8 à 13 pour déterminer la concentration en protamine-1 et en protamine-2 et le rapport entre la protamine-1 et la protamine-2 comme ratio protamine-protéine dans un échantillon mis en cause d'un individu à examiner afin de déterminer la fertilité des spermatozoïdes in vitro.
